# EUROPEAN PATENT SPECIFICATION

(11) **EP 2 275 161 B1**
(45) Date of publication and mention of the grant of the patent: **18.07.2012**
(21) Application number: 09009162.0
(22) Date of filing: 14.07.2009
(51) Int. Cl.: A61M 16/08, A61M 16/04

(54) **Changeable connection assembly for a closed suction tube**
Austauschbare Verbindungsanordnung für ein verschlossenes Ansaugtubus
Ensemble de connexion changeable pour tube d'aspiration fermé

(43) Date of publication of application: 19.01.2011
(73) Proprietor: Chang, Ti-Li, Houli Hsiang T'ai chung (TW)
(72) Inventor: Chang, Ti-Li, Houli Hsiang T'ai chung (TW)
(74) Representative: Schildberg, Peter

(56) References cited:
- WO-A-02/28463
- WO-A-96/26757
- WO-A-2006/133882
- US-A- 5 735 271
- US-A- 5 813 402

## Description

### 1. Field of the Invention

The present invention relates to a connection assembly, and more particularly to a changeable connection assembly for a closed suction tube.

### 2. Description of Related Art

A closed suction tube is used to draw secretion from a patient with a drawing force provided by a hospital vacuum source. Furthermore, the closed suction tube is used to perform the secretion suction on patient while providing ventilation simultaneously. Consequently, this can reduce bad influences to be vital sign of the patient.

However, the conventional connection assembly for a changeable closed suction tube does not have any sealing effect to a used closed suction tube, so secretion mounted on the closed suction tube may spread out thereof. Therefore, medical personnel easily contact with and are inflected by the secretion, and this easily causes cross inflection between patients and medial personnel in a hospital.

In addition, the conventional connection assembly is connected to a closed suction tube in a thread manner, so to connect and disconnect the closed suction tube to/from the conventional connector assembly is inconvenient.

WO 96/26757 and US 5,813,402 also disclose conventional connection assemblies for closed suction tubes.

To overcome the shortcomings, the present invention tends to provide a connection assembly to mitigate or obviate the aforementioned problems.

The main objective of the invention is to provide a connection assembly for a closed suction tube and that is convenient in changing a used closed suction tube. The connection assembly has a body, a connecting end and a sealing base. The body is hollow and has a connection portion. The connection portion is hollow, is formed on the body and has a connection base. The connection base is formed on the connection portion and has a front face and two engaging channels. The front face has a top and a bottom. The engaging channels are defined respectively in the top and the bottom of the front face of the connection base and face to each other. The connecting end is detachably attached to the front face of the connection base, communicates with the connection portion and has a connection plate. The connection plate has two edges respectively held slidably in the engaging channels in the connection base. The sealing base is mounted detachably on the connection portion and has a sealing face, a guiding channel and a holding notch. The sealing face is flush with the front face of the connection base. The guiding channel is defined around the sealing face and communicates with the engaging channels in the connection base. The holding notch is defined in the sealing base and is mounted around and holding the connection portion.

Other objects, advantages and novel features of the invention will become more apparent from the following detailed description when taken in conjunction with the accompanying drawings.

### IN THE DRAWINGS

Fig. 1 is a perspective view of a first embodiment of a connection assembly for a closed suction tube in accordance with the present invention;
Fig. 2 is an exploded perspective view of the connection assembly in Fig. 1;
Fig. 3 is an exploded perspective view of the body and the connecting end of the connection assembly in Fig. 1;
Fig. 4 is a cross sectional top view of the connection assembly in Fig. 1;
Fig. 5 is an operational cross sectional top view of the connection assembly in Fig. 1 showing a used connecting end being changed with an unused one;
Fig. 6 is a perspective view of a second embodiment of a connection assembly for a closed suction tube in accordance with the present invention;
Fig. 7 is a front view of the connection assembly in Fig. 6; and
Fig. 8 is an operational front view of the connection assembly in Fig. 6 showing a used connecting end being changed with an unused one.

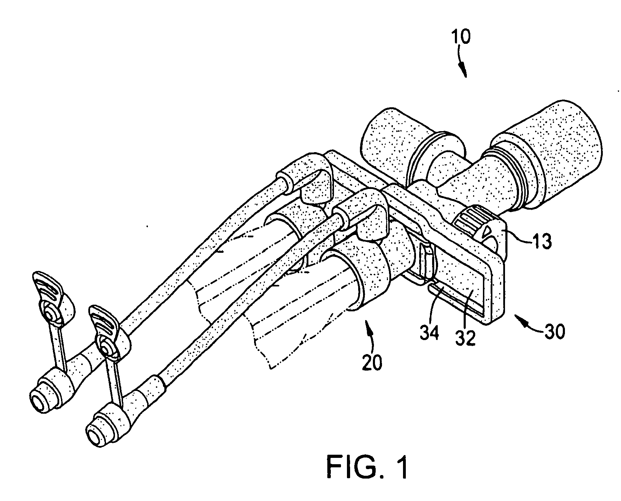

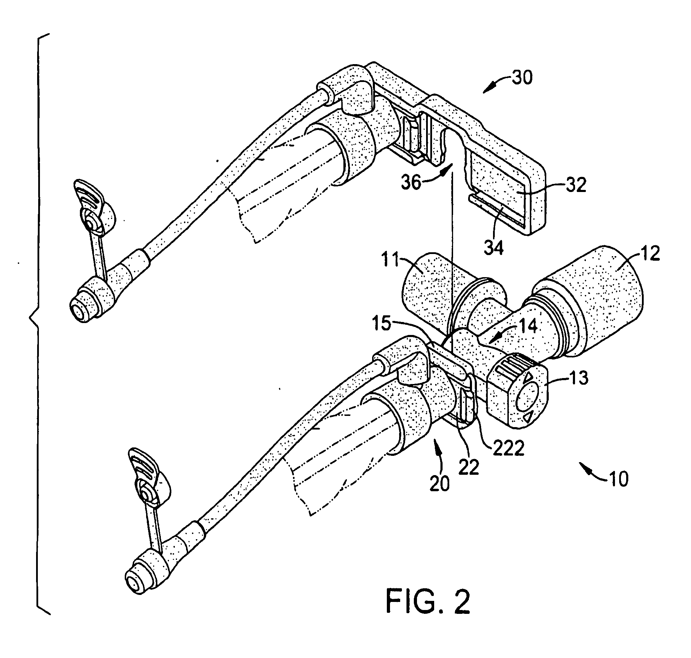

With reference to Figs. 1 to 3, a connection assembly for a closed suction tube in accordance with the present invention comprises a body (10), a connecting end (20) and a sealing base (30). The body (10) is hollow and comprises a respiratory tube connector (11), an airway device connector (12), a connection portion (14) and an isolation valve (13). The respiratory tube connector (11) and the airway device connector (12) are formed on the body (10), communicate with each other and are connected respectively to a respiratory tube system and an airway device that is connected to a patient. The connection portion (14) is hollow and formed on the body (10), communicates with the respiratory tube connector (11) and the airway device connector (12) and has a connection base (15). The connection base (15) is formed on the connection portion (14) and comprises a front face and two engaging channels (152). The front face has a top, a bottom and an opening (142). The opening (142) is defined through the front face and communicates with the connection portion (14). The engaging channels (152) are defined respectively in the top and the bottom of the front face of the connection base (15) and face to each other. The isolation valve (13) is mounted on the body (10) to turn on or off a communication between the connection portion (14) and the connectors (11,12).

The connecting end (20) with a suction tube, is detachably attached to the front face of the connection base (15), communicates with the connection portion (14) via the opening (142) and comprises a connection plate (22). The connection plate (22) has two edges, a contacting face, an annular groove, an O-ring (24) and a holding assembly. The edges are respectively held slidably in the engaging channels (152) in the connection base (15). The contacting face faces to and abuts with the front face of the connection base (15) when the connecting end (20) is connected to the connection base (15). The contacting face has an opening defined through the contacting face and aligning and communicating with the opening (142) in the connection base (15) when the connecting end (20) is connected to the connection base (15). The annular groove is defined in and around the opening in the contacting face. The O-ring (24) is mounted in the annular groove, abuts against the front face of the connection base (15) and is mounted around the opening (142) in the connection base (15). With the arrangement of the O-ring (24), a sealing effect is provided to the communication between the connection portion (14) and the connecting end (20) to prevent the leakage of secretion drawn out from a patient.

The holding assembly is formed on the connection plate (22) and engages the connection base (15) on the body (10) to securely hold the connecting end (20) on the connection base (15). In the first embodiment, the holding assembly comprises two resilient holding arms (222) formed respectively on two sides of the connection plate (22) and abutting respectively with two sides of the connection base (15). With the abutment between the resilient holding arms (222) with the sides of the connection base (15), the connection plate (22) is kept from sliding relative to the engaging channels (152) so that the connecting end (20) will not be detached from the connection base (15) unintentionally.

The sealing base (30) is mounted detachably on the connection portion (14) and comprises a sealing face (32), a guiding channel (34) and a holding notch (36). The sealing face (32) is flush with the front face of the connection base (15) when the sealing base (30) is mounted on the connection portion (14). The guiding channel (34) is defined around the sealing face (32) and communicates with the engaging channels (152) in the connection base (15). The holding notch (36) is defined in the sealing base (30) and is mounted around and holds the connection portion (14) to connect the sealing base (30) onto the body (10). The holding notch (36) is defined in a middle portion of the sealing base (30) to formed two sealing zones on the sealing face (32) respectively at two sides of the holding notch (36). In practice, the sealing base (30) may further have a connecting end (20) mounted slidably on one of the sealing zones on the sealing face (32).

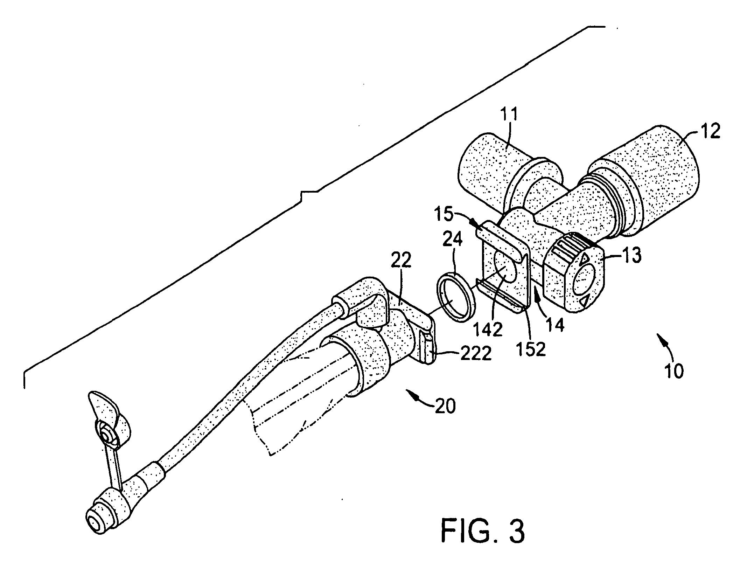

In use, with reference to Fig. 3, the body (10) is connected to a respiratory tube system and an airway device with the respiratory tube connector (11) and the airway device connector (12), and the connecting end (20) is connected to the suction tube. The connecting end (20) is connected to the connection portion (14) of the body (10) with the edges of the connection plate (22) being held respectively in the engaging channels (152) in the connection base (15). Accordingly, secretion, such as phlegm can be drawn out from a patient via the airway device, the body (10), the connecting end (20) and the suction tube with drawing force provided by a hospital vacuum source and a suction control valve in the end of tube.

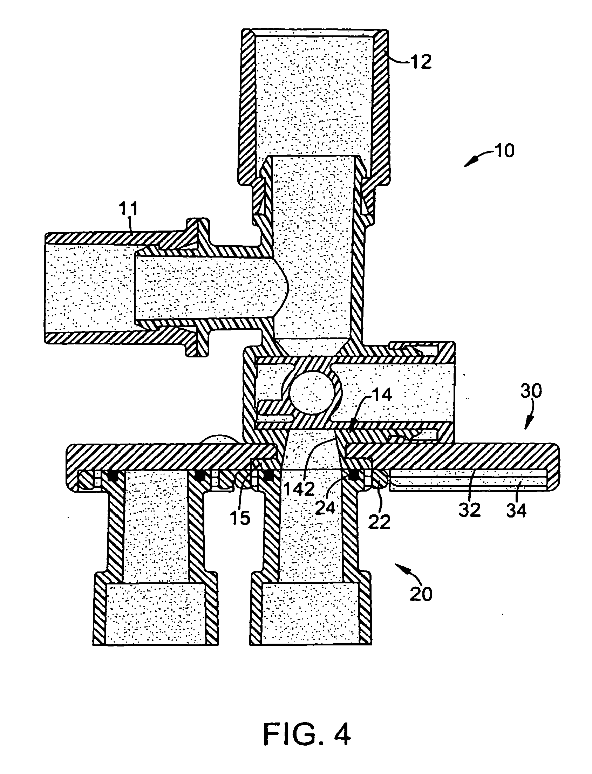

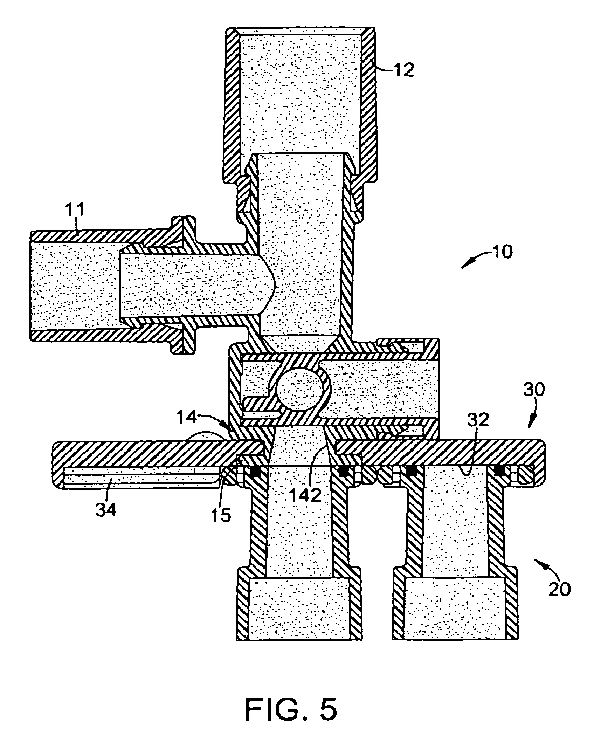

To change a used suction tube, with reference to Figs. 4 and 5, the isolation valve (13) is turned off firstly. The sealing base (30) is then attached to the connection portion (14) on the body (10) to make the sealing face (32) being flush with the front face of the connection base (15) and the guiding channel (34) aligning and communicating with the engaging channels (152). At this time, the sealing face (32) of the sealing base (30) will push against the resilient holding arms (222) on the connection plate (22) to disengage from the sides of the connection base (15). Consequently, the connecting end (20) that is connected to the used suction tube can be moved away from the connection base (15) and to one of the sealing zones along the engaging channels (152) and the guiding channel (34). The sealing base (30) is then detached from the connection portion (14), and the used connecting end (20) and the used suction tube can be disconnected from the body (10). With the sealing face (32) on the sealing base (30), the connecting end (20) can be well sealed to prevent the secretion in the closed suction tube from leaking therefrom. Therefore, medical personnel can be kept from contacting with and being inflected by the secretion, such that cross inflection between patients and medical personnel can be efficiently avoided.

If a new connecting end (20) with an unused suction tube is mounted previously on the sealing base (30), the new and unused connecting end (20) can be moved toward and engaged the connection base (15) and pushes the used connecting end (20) to move away from the connection base (15). After the sealing base (30) being detached from the body (10), new and unused connecting end (20) and suction tube are connected to the body (10). Accordingly, the process for replacing a new closed suction tube is easy and convenient.

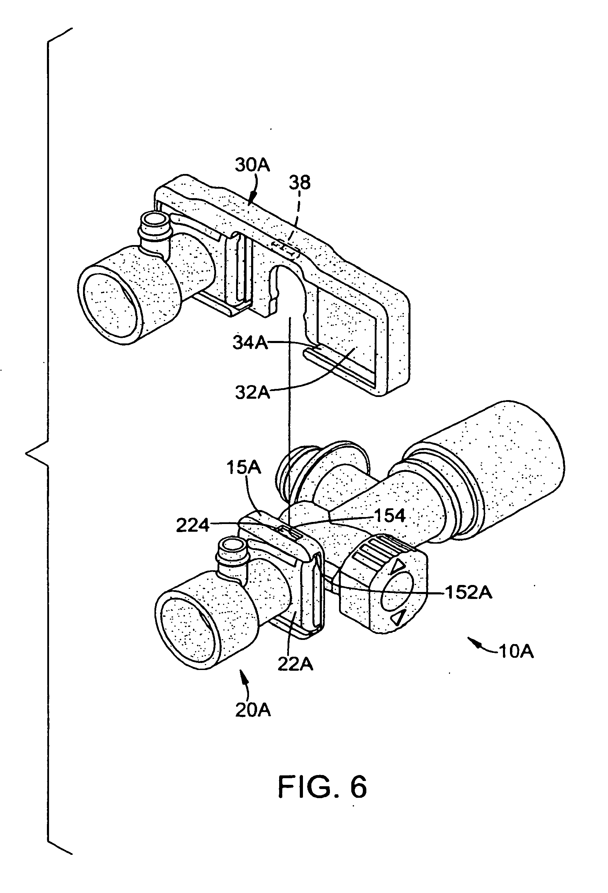

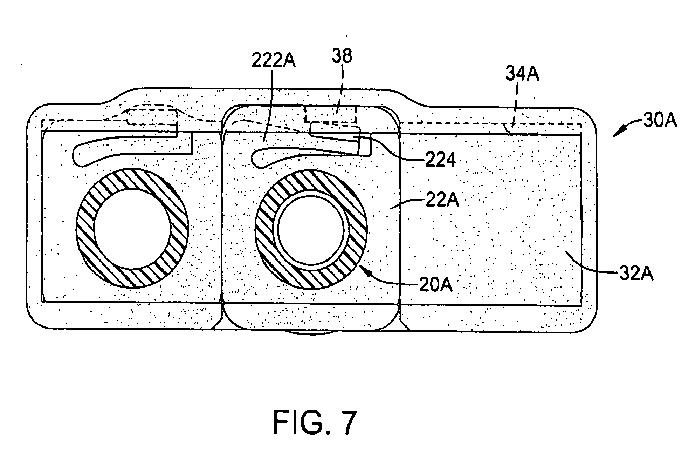

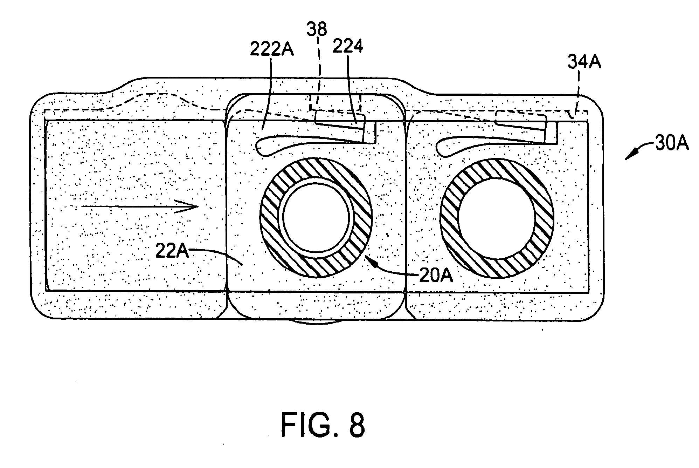

With reference to Figs. 6 to 8, in a second embodiment of a connection assembly in accordance with the present invention, the holding assembly on the connection plate (22A) comprises a resilient holding arm (222A) formed on one of the edges of the connection plate (22A) that engages one of the engaging channels (152A) in the connection base (15A). The resilient holding arm (222A) has an engaging block (224) formed on the resilient holding arm (222A). The connection base (15A) further has an engaging hole (154) defined in the connection base (15A) and engaging the engaging block (224) on the resilient holding arm (222A). With the engagement between the engaging block (224) on the resilient holding arm (222A) and the engaging hole (154), the connecting end (20A) will not be disconnected from the connection base (15A) unintentionally. Furthermore, the sealing base (30A) further has a pushing block (38) formed in the guiding channel (34A). When the sealing base (30A) is attached to the body (10A), the pushing block (38) is inserted into the engaging hole (154) and disengages the engaging block (224) on the resilient holding arm (222A) from the engaging hole (154). Accordingly, the connection plate (22A) is allowed to slide along the engaging channels (152A) and into the guiding channel (34A) and be sealed by the sealing face (32A) on the sealing base (30A).

## Claims

1. A connection assembly for a closed suction tube comprising:
a body (10,10A) being hollow and comprising a connection portion (14) being hollow, formed on the body (10,10A) and having a connection-base (15,15A) formed on the connection portion (14) and comprising
a front face having a top and a bottom; and
two engaging channels (152,152A) defined respectively in the top and the bottom of the front face of the connection base (15,15A) and facing to each other;
a connecting end (20,20A) communicating with the connection portion (14) and comprising a connection plate (22,22A) having two edges respectively held slidably in the engaging channels (152,152A) in the connection base (15,15A); and
a sealing base (30,30A) having
a sealing face (32,32A) being flush with the front face of the connection base (15,15A) when the sealing base (30,30A) is attached to the connection portion 14); and
a guiding channel (34,34A) defined around the sealing face (32,32A), **characterized in that**
the connecting end (20,20A) is adapted to connecting with suction tube and is detachably attached to the front face of the connection base (15,15A);
the sealing base (30,30A) is mounted detachably on the connection portion (14);
the guiding channel (34,34A) communicates with the engaging channels (152,152A) in the connection base (15,15A); and
the sealing base (30,30A) further has
a holding notch (36) defined in the sealing base (30,30A) and the holding notch (36) is mounted around and held by the connection portion (14).

2. The connection assembly as claimed in claim 1, wherein
the connection plate (22,22A) on the connecting end (20,20A) has a holding assembly formed on the connection plate (22,22A) and engaging the connection base (15,15A) on the body (10, 10A).

3. The connection assembly as claimed in claim 2, wherein the holding assembly on the connection plate (22) comprises two resilient holding arms (222) formed respectively on two sides of the connection plate (22) and abutting respectively with two sides of the connection base (15) after the sealing base (30) being detached from the connection portion (14) of the body (10).

4. The connection assembly as claimed in claim 2, wherein
the holding assembly on the connection plate (22A) comprises a resilient holding arm (222A) formed on one of the edges of the connection plate (22A) that engages one of the engaging channels (152A) in the connection base (15A) and having an engaging block (224) formed on the resilient holding arm (222A); and
the connection base (15A) further has an engaging hole (154) defined in the connection base (15A) and engaging the engaging block (224) on the resilient holding arm(222A).

5. The connection assembly as claimed in claim 4, wherein the sealing base (30A) further has a pushing block (38) formed in the guiding channel (34A) and the pushing block (38) is inserted into the engaging hole (154) to disengage the engaging block (224) on the resilient holding arm (222A) from the engaging hole (154) when the sealing base (30A) is attached to the connection portion (14) of the body (10A).

6. The connection assembly as claimed in one of claims 1 to 5, wherein the connection plate (22,22A) of the connecting end (20,20A) has
a contacting face facing to and abutting with the front face of the connection base (15,15A);
an annular groove defined in the contacting face; and
an O-ring (24) mounted in the annular groove and abutting against the front face of the connection base (15,15A).

7. The connection assembly as claimed in claim 6, wherein the holding notch (36) is defined in a middle portion of the sealing base (30,30A) to form two sealing zones on the sealing face (32,32A) respectively at two sides of the holding notch (36).

8. The connection assembly as claimed in claim 7, wherein the sealing base (30,30A) further has the connecting end (20,20A) mounted slidably on one of the sealing zones on the sealing face (32,32A).

9. The connection assembly as claimed in one of claims 1 to 5, wherein the holding notch (36) is defined in a middle portion of the sealing base (30,30A) to form two sealing zones on the sealing face (32,32A) respectively at two sides of the holding notch (36).

10. The connection assembly as claimed in one of claims I to 5, wherein the sealing base (30,30A) further has the connecting end (20,20A) mounted slidably on one of the sealing zones on the sealing face (32,32A).

## Patentansprüche

1. Verbindungsanordnung für ein verschlossenes Absaugtubus, umfassend:
einen Körper (10, 10A), der hohl ist und einen Verbindungsabschnitt (14) umfasst, der hohl ist, an dem Körper (10, 10A) geformt ist und einen Verbindungsträger (15, 15A) aufweist, der an dem Verbindungsabschnitt (14) geformt ist und Folgendes umfasst
eine Vorderseite mit einer Oberseite und einer Unterseite, und
zwei Eingriffskanäle (152, 152A), die in der Oberseite beziehungsweise Unterseite der Vorderseite des Verbindungsträgers (15, 15A) bestimmt und einander zugewandt sind;
ein Verbindungsende (20, 20A), das mit dem Verbindungsabschnitt (14) in Verbindung steht und eine Verbindungsplatte (22, 22A) umfasst, die zwei Kanten aufweist, die jeweils verschiebbar in den Eingriffskanälen (152, 152A) in dem Verbindungsträger (15, 15A) gehalten sind; und
einen Dichtträger (30, 30A), aufweisend
eine Dichtfläche (32, 32A), die bündig mit der Vorderseite des Verbindungsträgers (15, 15A) abschließt, wenn der Dichtträger (30, 30A) an dem Verbindungsabschnitt (14) befestigt ist; und
einen Führungskanal (34, 34A), der um die Dichtfläche (32, 32A) herum bestimmt ist, **dadurch gekennzeichnet, dass**
das Verbindungsende (20, 20A) an das Verbinden mit dem Absaugtubus angepasst ist und abnehmbar an der Vorderseite des Verbindungsträgers (15, 15A) befestigt ist;
der Dichtträger (30, 30A) abnehmbar an dem Verbindungsabschnitt (14) montiert ist;
der Führungskanal (34, 34A) mit den Eingriffskanälen (152, 152A) in dem Verbindungsträger (15, 15A) in Verbindung steht; und
der Dichtträger (30, 30A) ferner Folgendes aufweist
eine Haltekerbe (36), die in dem Dichtträger (30, 30A) bestimmt ist, und wobei die Haltekerbe (36) um den Verbindungsabschnitt (14) herum montiert ist und von diesem gehalten wird.

2. Verbindungsanordnung nach Anspruch 1, wobei die Verbindungsplatte (22, 22A) an dem Verbindungsende (20, 20A) eine Halteanordnung aufweist, die an der Verbindungsplatte (22, 22A) geformt ist und in den Verbindungsträger (15, 15A) an dem Körper (10, 10A) eingreift.

3. Verbindungsanordnung nach Anspruch 2, wobei die Halteanordnung an der Verbindungsplatte (22) zwei elastische Haltearme (222) umfasst, die jeweils an zwei Seiten der Verbindungsplatte (22) geformt sind und jeweils an zwei Seiten des Verbindungsträgers (15) angrenzen, nachdem der Dichtträger (30) von dem Verbindungsabschnitt (14) des Körpers (10) gelöst wurde.

4. Verbindungsanordnung nach Anspruch 2, wobei die Halteanordnung an der Verbindungsplatte (22A) einen elastischen Haltearm (222A) umfasst, der an einer der Kanten der Verbindungsplatte (22A) geformt ist und in einen der Eingriffskanäle (152A) in dem Verbindungsträger (15A) eingreift und einen Eingriffsblock (224) aufweist, der an dem elastischen Haltearm (222A) geformt ist, und
der Verbindungsträger (15A) ferner ein Eingriffsloch (154) aufweist, das in dem Verbindungsträger (15A) bestimmt ist und mit dem Eingriffsblock (224) an dem elastischen Haltearm (222A) in Eingriff steht.

5. Verbindungsanordnung nach Anspruch 4, wobei der Dichtträger (30A) ferner einen Druckblock (38) aufweist, der in dem Führungskanal (34A) geformt ist und der Druckblock (38) in das Eingriffsloch (154) gesteckt wird, um den Eingriffsblock (224) an dem elastischen Haltearm (222A) aus dem Eingriffsloch (154) zu lösen, wenn der Dichtträger (30A) an dem Verbindungsabschnitt (14) des Körpers (10A) befestigt wird.

6. Verbindungsanordnung nach einem der Ansprüche 1 bis 5, wobei die Verbindungsplatte (22, 22A) des Verbindungsendes (20, 20A) aufweist
eine Kontaktfläche, die der Vorderseite des Verbindungsträgers (15, 15A) zugewandt ist und an sie anstößt,
eine ringförmige Rille, die in der Kontaktfläche bestimmt ist, und
einen O-Ring (24), der in der ringförmigen Rille montiert ist und an die Vorderseite des Verbindungsträgers (15, 15A) anstößt.

7. Verbindungsanordnung nach Anspruch 6, wobei die Haltekerbe (36) in einem mittleren Abschnitt des Dichtträgers (30, 30A) bestimmt ist, um zwei Dichtungsbereiche an der Dichtfläche (32, 32A) jeweils auf zwei Seiten der Haltekerbe (36) zu bilden.

8. Verbindungsanordnung nach Anspruch 7, wobei ferner das Verbindungsende (20, 20A) verschiebbar an einem der Dichtungsbereiche an der Dichtfläche (32, 32A) des Dichtträgers (30, 30A) montiert ist.

9. Verbindungsanordnung nach einem der Ansprüche 1 bis 5, wobei die Haltekerbe (36) in einem mittleren Abschnitt des Dichtträgers (30, 30A) bestimmt ist, um zwei Dichtungsbereiche an der Dichtfläche (32, 32A) jeweils auf zwei Seiten der Haltekerbe (36) zu bilden.

10. Verbindungsanordnung nach einem der Ansprüche 1 bis 5, wobei ferner das Verbindungsende (20, 20A) verschiebbar an einem der Dichtungsbereiche an der Dichtfläche (32, 32A) des Dichtträgers (30, 30A) montiert ist.

## Revendications

1. Ensemble de connexion pour un tube d'aspiration fermé, comprenant :
un corps (10, 10A) creux comprenant une portion de connexion (14) creuse formée sur le corps (10, 10A) et ayant une base de connexion (15, 15A) formée sur la portion de connexion (14) et comprenant
une face frontale ayant un haut et un bas ; et
deux canaux de mise en prise (152, 152A) définis respectivement dans le haut et le bas de la face frontale de la base de connexion (15, 15A) et se faisant face ;
une extrémité de connexion (20, 20A) communiquant avec la portion de connexion (14) et comprenant une plaque de connexion (22, 22A) ayant deux bords respectivement retenus de façon coulissante dans les canaux de mise en prise (152, 152A) dans la base de connexion (15, 15A) ; et
une base d'étanchéité (30, 30A) ayant
une face d'étanchéité (32, 32A) en affleurement avec la face frontale de la base de connexion (15, 15A) quand la base d'étanchéité (30, 30A) est attachée à la portion de connexion (14) ; et
un canal de guidage (34, 34A) défini autour de la face d'étanchéité (32, 32A),
**caractérisé en ce que**
l'extrémité de connexion (20, 20A) est adaptée pour se connecter au tube d'aspiration et est attachée de façon détachable à la face frontale de la base de connexion (15, 15A);
la base d'étanchéité (30, 30A) est montée de façon détachable sur la portion de connexion (14) ;
le canal de guidage (34, 34A) communique avec les canaux de mise en prise (152, 152A) dans la base de connexion (15, 15A) ; et
la base d'étanchéité (30, 30A) a également
une encoche de retenue (36) définie dans la base d'étanchéité (30, 30A), et l'encoche de retenue (36) est montée autour et retenue par la portion de connexion (14).

2. Ensemble de connexion selon la revendication 1, dans lequel
la plaque de connexion (22, 22A) sur l'extrémité de connexion (20, 20A) a un ensemble de retenue formé sur la plaque de connexion (22, 22A) et entrant en prise avec la base de connexion (15, 15A) sur le corps (10, 10A).

3. Ensemble de connexion selon la revendication 2, dans lequel
l'ensemble de retenue sur la plaque de connexion (22) comprend deux bras de retenue (222) résilients formés respectivement sur deux côtés de la plaque de connexion (22) et venant en butée respectivement avec deux côtés de la base de connexion (15) après le détachement de la base d'étanchéité (30) par rapport à la portion de connexion (14) du corps (10).

4. Ensemble de connexion selon la revendication 2, dans lequel
l'ensemble de retenue sur la plaque de connexion (22A) comprend un bras de retenue (222A) résilient formé sur un des bords de la plaque de connexion (22A) qui entre en prise avec un des canaux de mise en prise (152A) dans la base de connexion (15A) et ayant un bloc de mise en prise (224) formé sur le bras de retenue (222A) résilient ; et la base de connexion (15A) a également un trou de mise en prise (154) défini dans la base de connexion (15A) et entrant en prise avec le bloc de mise en prise (224) sur le bras de retenue (222A) résilient.

5. Ensemble de connexion selon la revendication 4, dans lequel la base d'étanchéité (30A) a également un bloc de poussée (38) formé dans le canal de guidage (34A), et le bloc de poussée (38) est inséré dans le trou de mise en prise (154) pour libérer le bloc de mise en prise (224) sur le bras de retenue (222A) résilient hors du trou de mise en prise (154) quand la base d'étanchéité (30A) est attachée à la portion de connexion (14) du corps (10A).

6. Ensemble de connexion selon l'une des revendications 1 à 5, dans lequel la plaque de connexion (22, 22A) de l'extrémité de connexion (20, 20A) a
une face de mise en contact faisant face à, et venant en butée avec la face frontale de la base de connexion (15, 15A) ;
une gorge annulaire définie dans la face de mise en contact ; et
un joint torique (24) monté dans la gorge annulaire et venant en butée contre la face frontale de la base de connexion (15, 15A).

7. Ensemble de connexion selon la revendication 6, dans lequel l'encoche de retenue (36) est définie dans une portion médiane de la base d'étanchéité (30, 30A) pour former deux zones d'étanchéité sur la face d'étanchéité (32, 32A) respectivement des deux côtés de l'encoche de retenue (36).

8. Ensemble de connexion selon la revendication 7, dans lequel la base d'étanchéité (30, 30A) a également l'extrémité de connexion (20, 20A) montée de façon coulissante sur une des zones d'étanchéité sur la face d'étanchéité (32, 32A).

9. Ensemble de connexion selon l'une des revendications 1 à 5, dans lequel l'encoche de retenue (36) est définie dans une portion médiane de la base d'étanchéité (30, 30A) pour former deux zones d'étanchéité sur la face d'étanchéité (32, 32A) respectivement des deux côtés de l'encoche de retenue (36).

10. Ensemble de connexion selon l'une des revendications 1 à 5, dans lequel la base d'étanchéité (30, 30A) a également l'extrémité de connexion (20, 20A) montée de façon coulissante sur une des zones d'étanchéité sur la face d'étanchéité (32, 32A).
